# EUROPEAN PATENT SPECIFICATION

(11) **EP 1 352 151 B1**
(45) Date of publication and mention of the grant of the patent: **06.10.2004**
(21) Application number: 02703559.1
(22) Date of filing: 15.01.2002
(51) Int. Cl.: E21B 47/06, E21B 49/10

(54) **MEASURING THE IN SITU STATIC FORMATION TEMPERATURE**
IN-SITU-STATISCHE TEMPERATURMESSUNG EINER UNTERIRDISCHEN FORMATION
MESURE DE LA TEMPERATURE STATIQUE D'UNE FORMATION IN SITU

(30) Priority: 18.01.2001 EP 01200179; 03.07.2001 US 302982 P
(43) Date of publication of application: 15.10.2003
(73) Proprietor: SHELL INTERNATIONALE RESEARCH MAATSCHAPPIJ B.V., 2596 HR Den Haag (NL)
(72) Inventor: HASHEM, Mohamed, Naguib, New Orleans, LA 70139 (US)
(86) International application number: PCT/EP2002/000442
(87) International publication number: WO 2002/057595

(56) References cited:
- WO-A-96/12088
- WO-A-97/08424
- US-A- 3 417 827
- US-A- 4 535 843
- US-A- 5 361 836

## Description

The present invention relates to measuring the in situ static formation temperature in a well during a drilling stage. Ceasing circulation also refers to shutting-in the borehole. Ceasing circulation leaves the borehole filled with drilling mud, and the temperature of the drilling mud will in general differ from the temperature of the undisturbed formation.

One way of measuring this temperature is lowering a thermometer into the borehole filled with drilling mud and recording the temperature at a predetermined depth as a function of time (Δt) after ceasing circulation. The thermometer can be a self-contained recording temperature or a surface-recording thermometer. When the temperature is constant with time, one assumes that this constant temperature is the in situ static formation temperature. However, this method is time consuming.

An improvement of the above-described method is the following method. In this method the temperature is plotted versus log((tk+Δt)/Δt), wherein tk is the circulation time and Δt is the time after ceasing circulation. The result is extrapolated to log((tk+Δt)/Δt)is substantially equal to zero, which means that the circulation time is negligible compared to the time after ceasing circulation. The extrapolated temperature is then assumed to be the in situ static formation temperature.

However, mistakes in the circulation time could make a large effect on the extrapolated temperature. Such a mistake is easily made, for example when after completing a drilling stage the driller pulls the drill bit up a couple of hundreds of meters, and continues circulation for several hours to clean the bit before shutting in the borehole. In which case he will report a circulation time that is the sum of the circulation time needed to drill the particular stage and the time required for cleaning the drill bit. Whereas in order to evaluate the temperature record, the circulation time should be the circulation time needed to drill the particular stage.

It is an object of the present invention to provide a more accurate measurement of the in situ static temperature.

To this end the method of measuring the in situ static temperature of a formation traversed by a borehole according to the present invention comprises the steps as disclosed in independent claim 1.

US patent specification No. 4 535 843 discloses a method for obtaining a formation fluid sample using a downhole tool. The tool withdraws fluid from the formation and the resistivity of the withdrawn fluid is measured until constant. When the resistivity is constant the formation fluid is indicated as uncontaminated and is directed into a separate test chamber where the temperature is measured in order to allow calibration of an electrode system for measuring acidity and redox potential.

International Patent Application with Publication No. WO 97/08424 discloses a downhole tool and method for flowing fluids selectively from a reservoir to surface, and for obtaining dynamic reservoir data. While fluid is produced to surface the known tool can measure, inter alia, the temperature of the fluid.

The invention will now be described in more detail. The first step of the method of measuring the in situ static temperature of a formation traversed by a borehole according to the present invention is lowering to a predetermined position in the borehole a tool that comprises a central conduit having an inlet and being provided with a temperature sensor in contact with the fluid, means for analysing the fluid, and means for discharging fluid. The predetermined position can be the bottom of the borehole, or a position in a formation layer of which the in situ static formation temperature is to be measured. The tool is lowered into the borehole by means of for example a wireline.

Then an exclusive fluid communication is made between the formation and the inlet of the central conduit. In order to make the exclusive fluid communication, a probe is extended into the formation, wherein the outlet of the probe is in direct fluid communication with the inlet of the central conduit of the tool. Because the inlet of the probe is located in the formation, drilling mud present in the borehole cannot enter into the central conduit, an exclusive fluid communication is made between the formation and the inlet of the central conduit and thus the wellbore fluids are isolated.

Then formation fluid is allowed to pass through the central conduit. This is done with the aid of a pump, sucking the formation fluid via the probe into the central conduit and discharging the formation fluid from the central conduit. It will be understood that during drilling of the borehole, drilling mud will invade the formation. Therefore, when formation fluid is withdrawn, first drilling mud will be withdrawn, then a mixture of drilling mud and original formation fluid and finally uncontaminated formation fluid.

As it passes through the central conduit, the formation fluid is analysed to determine its composition.

Then the temperature is measured continuously until the formation fluid is substantially uncontaminated. Applicant has found that the temperature of the uncontaminated formation fluid is not just a temperature of a liquid, but it is precisely the in situ static formation temperature.

In practice both the temperature and the composition will be recorded, and the in situ static formation temperature is the temperature that belongs to the substantially uncontaminated formation fluid.

Because in the method of the present invention the temperature of the uncontaminated formation fluid is measured, this method is more accurate than the known methods.

Moreover, the method according to the present invention can be carried out with a tool that is used to take samples of the formation fluids. Such a tool is for example the Modular Dynamics Formation Tester tool from Schlumberger. This tool also contains an accurate thermometer used to calibrate a pressure sensor, and the output of the accurate thermometer can be used in the method of the present invention. Other suitable tools known in the art are the repeat dynamic tester from Halliburton and the reservoir characterization instrument from Western Atlas.

The method of the present invention can as well be applied in a cased borehole. In this case the step of lowering the tool into the borehole comprises two steps. At first a perforation set is made through the casing wall into the formation at a location where the temperature needs to be established, wherein the perforation set comprises at least one perforation extending into the formation layer. Then the tool is lowered into the cased borehole. The tool is further provided with an upper and a lower packer arranged at either side of the inlet of the central conduit, wherein the central conduit opens below the lower packer or above the upper packer, and wherein the distance between the upper and the lower packer is larger than the height of a perforation set.

Making an exclusive fluid communication then comprises setting the packers so that the perforation set is straddled between the packers.

## Claims

1. A method of measuring the in situ static temperature of a formation traversed by a borehole, which method comprises the steps of
a) lowering to a predetermined position in the borehole a tool that comprises a central conduit having an inlet and being provided with a temperature sensor in contact with the fluid, means for analysing the fluid, and means for discharging fluid;
b) making an exclusive fluid communication between the formation and the inlet of the central conduit;
c) allowing formation fluid to pass through the central conduit;
d) analysing the formation fluid; and
e) measuring the temperature continuously until the formation fluid is substantially uncontaminated, and determining the in situ static temperature of the formation as the temperature of the substantially uncontaminated formation fluid.

2. The method according to claim 1, wherein making an exclusive fluid communication between the formation and the inlet of the central conduit comprises extending into the formation a probe having an outlet that is in direct fluid communication with the inlet of the central conduit of the tool.

3. The method according to claim 1, wherein the formation is traversed by a cased borehole, wherein step a) comprises
a1) making a perforation set through the casing wall into the formation at a location where the temperature needs to be established;
a2) lowering the tool into the borehole to the perforation set, which tool is further provided with an upper and a lower packer arranged at either side of the inlet of the central conduit, wherein the central conduit opens below the lower packer or above the upper packer, and wherein the distance between the upper and the lower packer is larger than the height of a perforation set, and wherein step b) comprises setting the packers so that the perforation set is straddled between the packers.

## Patentansprüche

1. Verfahren zum Messen der statischen in situ Temperatur einer Formation, die von einem Bohrloch durchsetzt ist, wobei das Verfahren die Schritte umfaßt:
a) Absenken eines Werkzeuges zu einer vorbestimmten Position in das Bohrloch, wobei das Werkzeug eine zentrale Leitung, die einen Einlaß hat und mit einem Temperaturfühler in Kontakt mit dem Fluid ausgestattet ist, Mittel zum Analysieren des Fluids, und Mittel zur Abgabe des Fluids aufweist;
b) Herstellen einer exklusiven Fluidverbindung zwischen der Formation und dem Einlaß der zentralen Leitung;
c) Zulassen, dass Formationsfluid durch die zentrale Leitung strömt;
d) Analysieren des Formationsfluids; und
e) kontinuierliches Messen der Temperatur, bis das Formationsfluid im wesentlichen unverschmutzt ist, und Ermitteln der statischen in situ Temperatur der Formation als Temperatur des im wesentlichen unverschmutzten Formationsfluids.

2. Verfahren nach Anspruch 1, bei welchem das Herstellen einer exklusiven Fluidverbindung zwischen der Formation und dem Einlaß der zentralen Leitung das Ausfahren eines Fühlers in die Formation umfaßt, der einen Auslaß hat, der in direkter Fluidverbindung mit dem Einlaß der zentralen Leitung des Werkzeuges steht.

3. Verfahren nach Anspruch 1, bei welchem die Formation von einem ausgekleideten Bohrloch durchsetzt ist, wobei der Schritt a) umfaßt:
a1) Herstellen eines Perforationssatzes durch die Auskleidungswand in die Formation an einer Stelle, an welcher die Temperatur festgestellt werden muß;
a2) Absenken des Werkzeuges in das Bohrloch zu dem Perforationssatz, wobei das Werkzeug ferner mit einem oberen und einem unteren Dichtungsstück versehen ist, die zu beiden Seiten des Einlasses der zentralen Leitung angeordnet sind, wobei die zentrale Leitung unterhalb des unteren Dichtungsstückes und oberhalb des oberen Dichtungsstückes ausmündet, und wobei der Abstand zwischen dem oberen und dem unteren Dichtungsstück größer als die Höhe eines Perforationssatzes ist, und wobei der Schritt b) das Einstellen der Dichtungsstücke umfaßt, derart, daß der Perforationssatz sich zwischen den Dichtungsstücken erstreckt.

## Revendications

1. Procédé de mesure de la température statique in situ d'une formation traversée par un sondage, lequel procédé comprend les étapes suivantes :
a) l'abaissement à une position prédéterminée dans le sondage d'un outil qui comprend un conduit central comportant une entrée et étant pourvu d'un capteur de température en contact avec le fluide, d'un moyen pour analyser le fluide, et d'un moyen pour évacuer le fluide;
b) la réalisation d'une communication pour un fluide exclusive entre la formation et l'entrée du conduit central;
c) le passage du fluide de la formation par le conduit central;
d) l'analyse du fluide de la formation; et
e) la mesure en continu de la température jusqu'à ce que le fluide de la formation soit sensiblement non contaminé, et la détermination de la température statique in situ de la formation comme température du fluide de la formation sensiblement non contaminé.

2. Procédé suivant la revendication 1, dans lequel la réalisation d'une communication pour un fluide exclusive entre la formation et l'entrée du conduit central comprend le déploiement dans la formation d'une sonde comportant une sortie qui est en communication pour un fluide direct avec l'entrée du conduit central de l'outil.

3. Procédé suivant la revendication 1, dans lequel la formation est traversée par un sondage tubé, dans lequel l'étape a) comprend :
a1) la réalisation d'une série de perforations à travers la paroi du tubage dans la formation en un emplacement où la température doit être établie;
a2) l'abaissement de l'outil dans le sondage jusqu'à la série de perforations, lequel outil est de plus pourvu d'un packer supérieur et inférieur agencés de chaque côté de l'entrée du conduit central, dans lequel le conduit central s'ouvre en dessous du packer inférieur et au-dessus du packer supérieur, et dans lequel la distance entre le packer supérieur et inférieur est plus grande que la hauteur d'une série de perforations, et dans lequel l'étape b) comprend la mise en place des packers de telle sorte que la série de perforations soient positionnées entre les packers.
